(19) Europäisches Patentamt

European Patent Office

Office européen des brevets

(11) **EP 3 467 689 A1**

(12) **EUROPEAN PATENT APPLICATION**
published in accordance with Art. 153(4) EPC

(43) Date of publication:
**10.04.2019 Bulletin 2019/15**

(21) Application number: **16904507.7**

(22) Date of filing: **14.12.2016**

(51) Int Cl.:
**G06F 19/12** (2011.01)

(86) International application number:
**PCT/CN2016/109895**

(87) International publication number:
**WO 2017/211059 (14.12.2017 Gazette 2017/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **07.06.2016 CN 201610399510**

(71) Applicants:
• **Wang, Zhong**
  **Beijing 100700 (CN)**

• **Li, Bing**
  **Beijing 100700 (CN)**

(72) Inventors:
• **Wang, Zhong**
  **Beijing 100700 (CN)**
• **Li, Bing**
  **Beijing 100700 (CN)**

(74) Representative: **Schiweck Weinzierl Koch**
**Patentanwälte Partnerschaft mbB**
**Ganghoferstraße 68 B**
**80339 München (DE)**

(54) **METHOD FOR DIFFERENTIATING OR COMPARING DRUG ACTIVITY MODULE**

(57) Provided is a method for differentiating or comparing a drug activity module. The method comprising the following steps: (1) performing module categorization of two biological networks to obtain a modular set; (2) calculating one or more topological characteristics of two modular sets to obtain a single or combined topological indicator, and using the topological indicator to calculate a topological parameter change of a module corresponding to the two modular sets; and (3) comparing the topological parameter change of the module and a significance threshold; if the two biological networks are respectively a pre-drug intervention biological network and a post-drug intervention biological network, and if the topological parameter change is greater or equal to the significance threshold, then categorizing the module as a drug activity module; if the two biological networks are respectively a post-drug intervention biological network for a first drug and a post-drug intervention biological network for a second drug, and if the topological parameter change is greater or equal to the significance threshold, then categorizing the module as an activity difference module between a first drug and a second drug.

**Figure 1**

## Description

## Technical Field

The present invention belongs to bioinformatics. Specifically, the present invention relates to methods of identifying and comparing drug target modules based on biological networks.

## Background of the Invention

The emerging of Systems Biology and Network Pharmacology has made drug mechanism research and new drug R&D developed from mode of "Single Component, Single Target and Single Disease" to that of "Multiple Components, Multiple targets and Multiple Pathways". Studying diseases, drugs, target data and interactions thereof in the context of network, with integration of high-throughput data such as genome, transcriptome, proteome and metabolome data and combination of various mathematical models and algorithms, has also been a hot spot in the research of disease-drug interaction mechanism.

The occurrence and development of many complex diseases are related to a series of interacting genes or proteins, while disease phenotypes are manifestations of different biological processes interacting in a complex network. A drug, by acting on multiple targets in a disease network, exerting a synergistic effect on the targets, and thereby intervening the occurrence and development of the disease, ultimately achieves a therapeutic effect. Therefore, in the field of disease research and drug development, network analysis methods are increasingly utilized to identify biological markers of complex diseases, targets of drugs and the like. The analysis of biological networks also provides a new approach to a better understanding of the pathological mechanism of complex diseases, a systematical revealing of the pharmacological mechanism of drugs, and an effective development of new drugs.

Modularity is one of important characteristics of Systemic Biological Network. In the context of Systemic Biological Network, modularly analyzing a network by various calculating models and algorithms as well as deconstructing and analyzing the network at a modular level have become a new research direction of Network Pharmacology and Systems Biology. Module is a tightly connected functional group in a biological network, which is also expressed as cluster, subnetwork, community, subset, motif and the like in literatures. Module exhibits topological characteristics of close connection in a biological network, as well as stability and functionality. On the basis of principles of network clustering, heuristic search, seed extension, matrix decomposition, topology network and the like, researchers have developed many methods for module division, thus research on modularizing has become a hot spot in the research of biological network.

Pathological mechanism underlying diseases and action mechanism of drugs also feature modularization. The drug discovery model of "Single component and Single target" ignores the interaction between target genes or proteins. Therefore, certain researches have been tried to screen drugs with specific targets and lower side effects from a perspective of modularization. The conceptual framework of Modular Pharmacology (MP) has also been proposed, which is to reveal the relationship between drugs and diseases from the perspective of modularization, and to treat complex diseases with multi-targeted and modularization-designed drugs. This approach, which targets a module rather than independent genes or proteins, provides a new strategy for understanding the action mechanism of drugs and developing new drugs. In this regard, how to identify target modules of drugs in complex biological networks becomes a challenge. Therefore, it is of great significance to explore methods for identifying drug target modules based on the characteristics of biological network modules such as topological structure, stability and functionality.

## Summary of the Invention

The present invention provides a method for identifying and comparing drug action modules based on biological networks. The method integrates topological structure analysis and statistical analysis of the modules to comprehensively identify action modules (also known as "response modules") of the biological networks after drug intervention, and in combination with biological functional analysis, identifies whether or not the modules are target modules; in addition, the method of the present invention can also be used for comparing and analyzing the differential responses of different drugs by comparing differential modules of biological networks after intervention by the drugs.

Definition

As used herein, the term "biological network" means a network composed of nodes and edges, which is represented by G(V, E), in which V represents a set of nodes in the network each of which can be any possible target of a drug such as gene, protein, compound or metabolite and the like; and E represents a set of edges in the network each of which is an interacting relationship between the nodes, for example, it can be gene co-expression or interaction relationship, protein interaction relationship, transcriptional regulation relationship, and the like. Common biological networks include gene co-expression network, protein interaction network, metabolic network, gene transcription regulatory network, non-coding gene regulatory network, and various cross-omics regulatory and interacting networks, etc.. Taking gene co-expression network as an example, the gene co-expression network is constructed based on the correlation of expression profile data between genes and often utilizes graph model to describe the relationship between genes, in which nodes represent genes while edges represent the co-expressing interaction relationships between two genes.

As used herein, the term "module" refers to a tightly connected functional group consisting of nodes and edges in a biological network, with meaning equivalent to "subset", "clustering", "community", "subnet", and "motif", etc. in a biological network.

As used herein, the term "drug action module" refers to a module whose topological parameters change significantly compared with the corresponding module in the biological network before drug intervention, which is used interchangeably with the term "drug response module".

As used herein, the term "drug target module" refers to a drug action module which is confirmed to have relevant functions through function enrichment.

As used herein, the term "drug differential module" refers to a module whose topological parameters change significantly compared with the corresponding module in the biological networks obtained after intervention by two drugs.

As used herein, the term "drug conservative module" refers to a module whose topological parameters change insignificantly compared with the corresponding module in the biological networks obtained after intervention by two drugs.

Technical solutions of the present invention are as follows in detail.

The present invention provides a method for identifying or comparing drug action module(s), which comprises the following steps:

(1) performing module division of a first biological network $G_i(V_i, E_i)$ and a second biological network $G_j(V_j, E_j)$ to obtain collections of modules $G_i(M_i)$ and $G_j(M_j)$ respectively; wherein the first biological network $G_i(V_i, E_i)$ is a biological network before drug intervention, and the second biological network $G_j(V_j, E_j)$ is a biological network after drug intervention; or the first biological network $G_i(V_i, E_i)$ is a biological network after intervention by a first drug, and the second biological network $G_j(V_j, E_j)$ is a biological network after intervention by a second drug;

(2) calculating one or more topological characteristics M(t) of each module in the collections of modules $G_i(M_i)$ and $G_j(M_j)$ to obtain a single or combined topological indicator $T= M(t_1, t_2...t_m)$, wherein m is the number of the topological characteristics, and calculating a topological parameter change $\Delta t/T$ of corresponding modules in the two collections of modules;

(3) comparing the topological parameter change $\Delta t/T$ of the corresponding modules with a significance threshold W, when the first biological network $G_i(V_i, E_i)$ is a biological network before drug intervention and the second biological network $G_jV_j, E_j)$ is a biological network after drug intervention, if $\Delta t/T$ is greater or equal to the significance threshold W, the module is a drug action module; when the first biological network $G_i(V_i, E_i)$ is a biological network after intervention by the first drug and the second biological network $G_j(V_j, E_j)$ is a biological network after intervention by the second drug, if $\Delta t/T$ is greater or equal to the significance threshold W, the module is a differential module between the actions of the first drug and the second drug.

Preferably, the method of the present invention may be used for identifying whether or not the drug action module is a drug target module, i.e., the method further comprises:

(4) performing biological function enrichment analysis of the drug action module to determine whether or not the module is a drug target module based on the results of function enrichment.

In the step (1) of the method of the present invention, the drug, the first drug and the second drug belong to the same type of traditional Chinese medicinal drug or chemical drug for prevention and treatment of disease. Preferably, the first drug and the second drug are bioactive ingredients of the same type of traditional Chinese medicinal drug or chemical drug.

In the first and the second biological networks, $V_i$ or $V_j$ is a set of nodes in the network, while $E_i$ or $E_j$ is a set of edges in the network. Preferably, the first biological network and second biological network are biological molecular networks, for example selected from the group consisting of gene co-expression network, protein interaction network, gene transcription regulatory network, non-coding gene regulatory network and metabolic network. More preferably, the first biological network and the second biological network are gene co-expression network or protein interaction network of the same type; more preferably, the number of nodes in the biomolecular network is greater or equal to 200, greater or equal to 500, greater or equal to 1000, or greater or equal to 5000.

Preferably, the module division is performed by utilizing a network analysis algorithm selected from the group consisting of clustering, heuristic search, seed extension, matrix decomposition, and topology network, etc.; more preferably, the module division is performed by utilizing a method selected from the group consisting of hierarchical clustering algorithm, WGCNA, MCODE, MCL, CFinder, CPM, SPC, G-N algorithm, ModuLand, DME, MINE, and SVD.

In the step (2) of the method of the present invention, the topological characteristic M(t) is selected from the group consisting of modularity, degree, connectivity, density, clustering coefficient, betweenness, diameter, shortest path, entropy and characteristic path length.

Preferably, the number of the topological characteristics m is in the range of 1 to 10, for example m may be any integer in the range of 1 to 10.

Preferably, the number of the topological characteristics m is 2, and the topological characteristic M(t) comprises density and connectivity; or preferably, the number of the topological characteristics m is 3, and the topological characteristic M(t) comprises density, connectivity and entropy.

Preferably, calculating the topological parameter change $\Delta t/T$ comprises:
calculating the change of each topological parameter of each module of the first biological network compared with that of the corresponding module of the second biological network, i.e. calculating $\Delta t/T$ value of each topological parameter, and normalizing the $\Delta t/T$ value of each topological parameter by Min-max normalization, after which the $\Delta t/T$ value of each topological parameter is between (0, 1).

In the step (3) of the method of the present invention, the significance threshold W of the change of topological parameter of module is calculated using permutation test or bootstrap resampling.

Preferably, the significance threshold W is obtained by means as follow:
obtaining more than 1000 random modules corresponding to each module of the first biological network by utilizing permutation test or bootstrap resampling, calculating the normalized $\Delta t/T$ value of each random module compared with the corresponding module in the second biological network, and in turn calculating the probability of normal distribution of the normalized $\Delta t/T$ values of all random modules, accordingly the significance threshold W equaling the corresponding normalized $\Delta t/T$ value when p is less or equal to 0.05 in the probability of normal distribution.

In the step (4) of the present invention, the biological function enrichment analysis of the drug action module is carried out to determine whether or not it is a drug target module. There are many tools for function enrichment analysis of module, among which suitable ones can be selected according to the specific conditions of the module. Based on the results of function enrichment, it can be determined whether the drug action module has corresponding biological functions. For example, biological function enrichment analysis can be performed through analyzing gene ontology (GO) function and/or pathway. Generally, p value of function enrichment is calculated according to hypergeometric distribution. Function enrichment of GO is to calculate the hypergeometric distribution of genes constituting the module in one (several) specific branch(es) in GO classification and return the p value of GO function enrichment of the module, a small p value indicating that the module is enriched in the GO. Similarly, the pathway enrichment analysis is to calculate the hypergeometric distribution of genes constituting the module in the same Pathway and return the p value of the enrichment of the module in the pathway, a small p value indicating that the module is enriched in the Pathway.

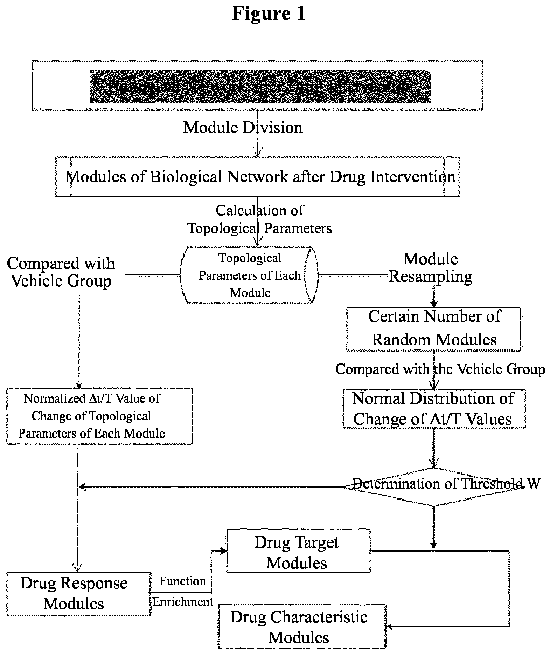

The schematic diagram of the method of the present invention is shown in Fig. 1.

There still lack effective methods for identifying and comparing drug action modules in biological networks, and how to determine the changes and significance thereof of modules in biological networks before and after drug intervention has always been a challenge. The present invention provides a method for identifying and comparing drug action and target modules by utilizing topological parameters of networks, and statistics and functional analysis methods, which can effectively discover drug action modules, compare the similarities and differences in action modules in case of different drugs, and provide a new strategy for drug research and development model using modules as drug targets.

Specifically, drug intervention will certainly have an impact on biological networks, and it is essential that how to determine which modules respond to drugs, that is, how to select target modules of the drugs. A module per se is also a small network composed of nodes and edges, so the module inherently owns topological features of a network, for example, the module characteristically has specific density, connectivity, shortest path, characteristic path length, and modularity, etc. Therefore, on the one hand, the method of the present invention utilizes the changes in the topological structures of the module in the network before and after intervention by a specific drug, identifies whether the module is a drug action module through comparison and analysis, and accordingly selects drug target modules by function enrichment analysis. On the other hand, the method of the present invention can utilize the similarities and differences in the topological structures of the module in the network after intervention by different drugs, determine the differential action modules of the different drugs and accordingly determine the similarities and differences in drug actions through comparison and analysis. Thus, the method of the present invention reveals the underlying mechanism of drug actions by observing the changes of the modular topological characteristics before and after drug intervention (rather than the changes in the expression level of independent genes and proteins).

## Description of the Figures

Hereinafter, the embodiments of the present invention will be described in detail with reference to the accompanying drawings, in which:

Fig. 1 shows the schematic diagram of the method of the present invention for identifying drug target module;

Fig. 2 shows the gene co-expression pattern of data of BA Group in Example 1;

Fig. 3 shows the gene co-expression pattern of BA Group in Example 1;

Fig. 4 shows the response modules (W>0.2) obtained through comparison of BA Group and Vehicle Group in Example 1;

Fig. 5 shows the differential modules (W>0.2) obtained through comparison of BA Group and JA Group in Example 1;

Fig. 6 shows module No. 16 of BA Group as an example and biological functions enriched in Example 1;

Fig. 7 shows target modules of BA Group as examples in Example 1;

Fig. 8 shows characteristic modules of BA Group as examples in Example 1;

Fig. 9 illustrates the interaction relationships of nodes in the BA drug target modules identified in Example 2 in a known database;

Fig. 10 demonstrates the expression of genes in the drug target modules identified in Example 2;

Fig. 11 illustrates the interaction relationships of genes in the drug target modules identified in Example 2;

Fig. 12 shows the result of module division of the effective group in Example 3;

Fig. 13 demonstrates the response modules of anti PD-1 treatment of melanoma through comparison of the effective group and the control group in Example 3;

Fig. 14 shows the result of module division of the effective group in Example 4;

Fig. 15 demonstrates the response modules of Infliximab treatment of RA through comparison of the effective group and the control group in Example 4.

## Best Modes for Carrying Out the Invention

The present invention will be further illustrated by the following particular Examples. Yet, it will be appreciated by those skilled in the art that the Examples described herein are only used to illustrate the present invention, and not to limit the scope of invention in any way.

Experimental methods in the following Examples are all conventional methods, if no any special instruction is provided. Raw materials of medicines and reagents used in the following Examples are all conventional products that are commercially available, if no any special instruction is provided.

**EXAMPLE 1** Identification of target modules in mouse model of cerebral ischemia

In this Example, gene expression profile data on model mice of cerebral ischemia which were intervened respectively with baicalin (BA), jasminoidin (JA) and ursodeoxycholic acid (UA), the bioactive ingredients extracted from Qingkailing, were used to construct gene co-expression networks before and after intervention by different drugs, and data from BA group were used to illustrate the implementation of the method of the present invention.

The schematic diagram of the whole implementing process is shown in Fig. 1.

1. Obtaining gene co-expression data

Baicalin (BA), jasminoidin (JA) and ursodeoxycholic acid (UA), the effective components of Qingkailing, were used for drug intervention in middle cerebral artery occlusion (MCAO) model in mice.

Construction of cerebral ischemia mouse model: MCAO model was established in mouse by slightly improved Zea-Longa Suture Embolization Method, through which the model of 1.5-hour cerebral ischemia followed by 24-hour reperfusion in mouse was prepared. A mouse fasted for 12 hours before operation but drank water freely. Then the mouse was anaesthetized by intraperitoneal injection of 10% chloral hydrate solution as anesthetic at a dose of 0.4 ml/100 g body weight, then placed in a supine position, made a midline incision on the neck to isolate and expose the left common carotid artery, external carotid artery and internal carotid artery. The common carotid artery and external carotid artery were carefully dissected with tweezers, with the vagus nerve separated. Afterwards, the common carotid artery was clamped with an artery clamp to temporarily block blood flow in the common carotid artery and the internal carotid artery. The distal end of external carotid artery was ligated with sterilized suture and the external carotid artery was cut, while ensuring that the length of the residual end of external carotid artery was longer than 0.5 cm. Extra suture was left at the proximal end of the internal carotid artery and an arterial clamp was applied at the distal end, then an incision was made at the enlarged bifurcation of the common carotid artery, the arterial clamp was loosen then, and 18-20 mm suture used for embolization was inserted into the internal carotid artery to block the middle cerebral artery at the beginning of the middle cerebral artery. Then the common carotid artery was ligated together with the suture with 10 mm free end left, finally the skin was sutured. The suture was withdrawn 1.5 hours after cerebral ischemia to make blood reflow. The neurological function of the mouse was scored after 24 h to determine whether the model was successfully established.

Established model mice of cerebral ischemia were randomly divided into 5 groups, including sham group, Vehicle group, BA group, JA group and UA group, in which treatment groups were injected BA (5 mg/ml), UA (7 mg/ml) and JA (25 mg/ml) via tail vein at a dose of 2 ml/kg body weight 2 h after cerebral ischemia respectively, and Sham group and Vehicle group were administered 0.9% NaCl via tail vein (2 ml/kg body weight). Then the RNA in hippocampus of each mouse was extracted by one-step method, and the quality of the RNA was evaluated using gene chips from Bioanalyzer. The gene expression profiles of mice after intervention were analyzed by a chip of Oligo fragments. The chip consisted of 374 c-DNAs related to cerebral ischemia. The gene expression profile data utilized in this Example were from four groups, i.e. Vehicle group (Vehicle), baicalin treatment group (BA), jasminoidin treatment group (JA) and ursodeoxycholic acid treatment group (CA).

The gene expression profile data of Vehicle group were taken as data before intervention, while those of BA group, JA group and UA group were taken as data after intervention. The gene expression profile data from each group were composed of 374 genes from 12 samples (Tbp, Zeb1, Pou2f1, Foxb1, Creb1, Camk2g, Csf1, F5, Hspd1, Matn2, Mt1, Adamts1, Klf6, Dffa, Rgs18, Rhoa, Kcnmb1, Pdcd11, Pdpk1, Casp8ap2, Mogat1, Rps26, Ak1, Csnk2a2, Dkk2, Ppmle, Tnfrsf22, Trp53i11, Smpd3, Grin1, Cdk5, Jund, E2f1, Apoe, Il1b, Prkarlb, Il7r, Ngfb, Rela, Ifnar1, Adcy6, Bak1, Fzd6, Prkch, Rgs4, Actg1, Gck, Rgs9, Sox9, Rgs1, Dgke, Rgs20, Map2k2, Pin1, Prkcn, Dgkz, Csnk1g1, Dusp4, Il11, Grb2, Shc1, Syk, Sim2, Ywhah, Fgfl3, Bid, Gstm2, Rarg, Pou3fl, Camk2b, Mapkapk2, Tcf4, Sos1, Stat5a, Vegfb, Bad, Etv3, Id1, Lcat, Nf1, Gsn, Bbc3, Clu, Capn9, Ercc5, Comt, Ctsl, Amph, Vegfc, Bax, Cyp51, Sox10, Nfyc, Gata2, Id3, Lef1, Pou6f1, 6330503C03Rik, Ech1, Ccl4, Itm2a, Hspala, Cbx3, Klf10, Idh3g, Gpx2, Map2k5, Daxx, E2f3, Fgf12, Ikbkg, Btrc, Ikbkap, Ifnar2, Cdk5, Psmb1, Sufu, Gab1, Sox30, Pxn, Pygo2, Ctnnb1, Grin2a, Il5ra, Cdk4, Bcl2l1, Actb, Myb, Prkca, Csf2rb2, Gnaq, B-raf, Wnt6, Adcy7, Cacnalb, Fzd7, Prkcm, Rock1, Adcy8, Prkcc, Sub1, Tubalb, Rgs6, Plcb1, Mknk1, Diablo, Mef2c, Lrplb, Dgkg, Rgs12, Serpina5, Hspb1, Ppmlb, Dlk1, Cdc42, Fadd, Mdfi, Fgf11, Map3k4, Klk1b3, Il6ra, Tgfb2, Wnt11, Ccna1, Map2k6, Htr1f, Zmat3, Bnip3, Tsg101, Vim, Srf, D14Abble, Cdh11, Vdac2, Tfdp1, Gak, Ccna2, Vegfa, Vegfa, Hdac1, Srebf1, Stch, E2fl, Nfatc1, Gna12, Gna13, Cacnb3, Zic1, Pou4f3, Tcfl2, Ldb1, Capns1, Fxyd2, Gcgr, LOC100304588, Syt11, Gadd45a, Pbx2, Ier3, Mapk9, Ctnnbip1, Fgf15, Smad3, Nlk, Mecp2, Sigirr, Rgs18, Ptk2b, Sap30bp, Pcmtl, Tcf3, Braf, Ankrd6, Rgs5, Raplgap, Adcy1, Grin2b, Gap43, Map2k1, Mapk10, Tgfb1, Lta, Rps6ka1, Wnt3, Rara, Prkcd, Atf4, Adcyap1r1, Cycs, Hint1, Rdx, Src, Adcy9, Prkce, Shcbpl, Elk3, Rgsl4, Rgsl7, Dusp10, Tubb3, Cyc1, Duspl6, Plcg2, Fzd10, Dgkd, Stat3, Mapkl4, Map2k4, Htrla, Map3k2, Fratl, Casp7, Eef2k, Thbd, Rarb, Camk4, Htr2c, E2f5, Met, Htr7, Camk2b, Stat6, Sod1, Efna4, Vdac3, Adora1, Bmpl, Vdac1, Grb2, Igfbp2, Top2b, Rpl35, Bdnf, Ppp3cb, Rafl, Cpe, Cacnb3, 0610007C21Rik, Gna14, Gna11, Tubala, Zic3, Mlx, Id4, Ldb2, Sepp1, Prodh, S100a9, Pgam2, Rcan1, Abcc5, Ccr5, Ap1m1, Map3k5, Csnkle, Axin1, Freq, Sh2b1, Rps6ka4, Wif1, Nkd1, Pam, Crem, Tgm2, Barhl1, Tradd, Plcd4, Ppp2r4, Otud7b, Rgs7, Casp2, Junb, Il2rg, Bad, Il1a, Egr1, Pdgfa, Gapdh, Eif4e, Apc, Prkcz, Parp1, Egfr, Prkcb1, Rgs2, Traf2, Ccr3, Rgs16, Smpd1, Tbp, Dgka, Mos, B230120H23Rik, Eif4e2, Rgs19, Adcy3, Creb5, Taf7, Pik3ca, Stat1, 1115, Atf3, Dvl3, Map3k3, Casp4, Kcnq1, Ptp4a3, fosB, Wnt3a, Calm1, Htr3a, Crkl, Casp3, Lhx1, Camk4, Selenbp2, Tcfe2a, Scg5, Pold3, Mmp2, Farp2, Pold2, Pold1, Gpx4, App, Mlh3, Rbl2, Tpp2, Cdh3, Fmo2, Pold4, Arf1, Sox1, and Arhgef1), and formed a 374×12 gene expression matrix. The format of some data elements is shown as follows:

Vehicle group:

| Array ID | Msam-1 | Msam-2 | Msam-3 | Msam-4 | Msam-5 | Msam-6 |
|---|---|---|---|---|---|---|
| Sc02R02C10 | 0.908 | 0.972 | 0.817 | 0.849 | 1.077 | 1.011 |
| Sc03R02C10 | 1.052 | 1.029 | 1.41 | 1.267 | 1.152 | 1.065 |
| Sc04R02C10 | 0.615 | 0.862 | 0.961 | 0.953 | 0.614 | 0.722 |
| Sc01R03C01 | 1.127 | 1.096 | 1.203 | 1.307 | 1.181 | 1.26 |
| Sc02R03C01 | 1.017 | 1.134 | 0.972 | 1.169 | 1.255 | 1.205 |
| Sc03R03C01 | 1.082 | 0.93 | 1.039 | 1.154 | 0.741 | 1.112 |
| Sc04R03C01 | 1.112 | 1.408 | 1.167 | 1.201 | 1.291 | 1.229 |
| Sc01R03C02 | 1.763 | 2.578 | 2.213 | 2.703 | 1.518 | 2.124 |

BA Group:

| Array ID | Sam 1 | Sam 2 | Sam 3 | Sam 4 | Sam 5 | Sam 6 |
|---|---|---|---|---|---|---|
| Sc02R02C10 | 1.019 | 0.966 | 1.019 | 1.121 | 1.146 | 0.967 |

(continued)

| Array ID | Sam 1 | Sam 2 | Sam 3 | Sam 4 | Sam 5 | Sam 6 |
|---|---|---|---|---|---|---|
| Sc03R02C10 | 0.79 | 0.948 | 1.106 | 0.758 | 1.016 | 0.995 |
| Sc04R02C10 | 0.931 | 1.003 | 0.873 | 0.712 | 1.02 | 0.833 |
| Sc01R03C01 | 1.089 | 1.137 | 1.368 | 1.326 | 1.282 | 0.991 |
| Sc02R03C01 | 1.269 | 1.482 | 1.451 | 1.522 | 1.044 | 1.321 |
| Sc03R03C01 | 1.21 | 0.625 | 1.082 | 0.903 | 0.83 | 0.876 |
| Sc04R03C01 | 1.822 | 2.376 | 0.804 | 1.226 | 2.714 | 1.944 |
| Sc01R03C02 | 1.782 | 2.199 | 1.929 | 2.252 | 4.277 | 4.575 |

The numerical value in each cell is the expression value of the gene (row) in the sample (column). According to the method of network construction, genes with the same expression pattern in the expression profile could be clustered together to form co-expression genes, which were clustered into a "gene co-expression module".

2. Network construction and module division

Gene co-expression networks (372 nodes for each network) of the three treatment groups were constructed utilizing weighted gene co-expression network analysis (WGCNA), then divided into modules. After module identification, totally 23 modules were obtained from BA group with module size ranging from 3 to 149 nodes (genes); totally 42 modules were obtained from JA group with module size ranging from 3 to 46 nodes; and totally 15 modules were obtained from UA group with module size ranging from 3 to 29 nodes (genes).

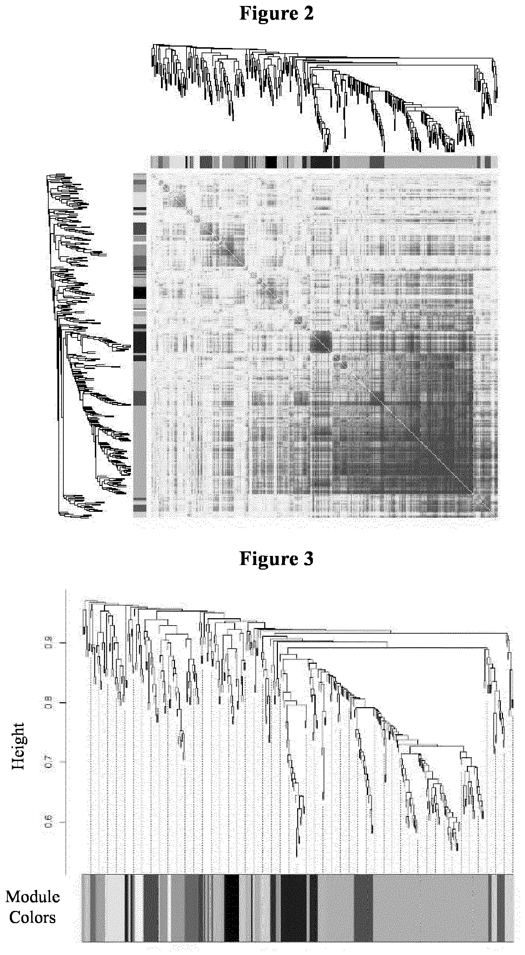

Taking modules of BA group as an example, the expression of all genes in the whole network was shown in Fig. 2, from which it was easy to identify the existence of co-expression modules. Modules of the BA group were obtained according to hierarchical clustering algorithm, each color representing a module, and the result was shown in Fig. 3.

3. Calculation of topological parameters of modules

The changes of topological characteristics of modules in different status before drug intervention (Vehicle group) and after intervention by BA (BA group) were calculated.

Density and connectivity were selected as topological indicators of the modules, and the density and connectivity of modules of BA intervention group and Vehicle group were calculated respectively. The BA intervention group was compared with the Vehicle group to observe the changes of density and connectivity between the two groups.

Density:

$$density = \mathrm{mean}(vectorize Matrix(A)).$$

Connectivity:

$$connectivity = \quad k_i = \sum_{j \neq i} a_{ij}.$$

4. Calculation of significance of the changes of topological characteristics

Significance of the changes of topological characteristics of the modules from the two groups was verified by Permutation test, in which 23 modules of BA group were randomly permutated 1000 times, the normalized $\Delta t/T$ value of each module compared with the Vehicle group was calculated, then accordingly the probability of normal distribution of the normalized $\Delta t/T$ values in the 1000 random permutations was obtained, and the correspondingly minimum value of the normalized $\Delta t/T$ values when p was less than or equal to 0.05 was the significance threshold of the change of topological parameters of the modules after drug intervention. When p was less than or equal to 0.05, W=0.2 was the significance threshold of the change of the modules. Compared with Vehicle group, the module with a $\Delta t/T$ value greater

than 0.2 in BA intervention group could be identified as a drug response module.

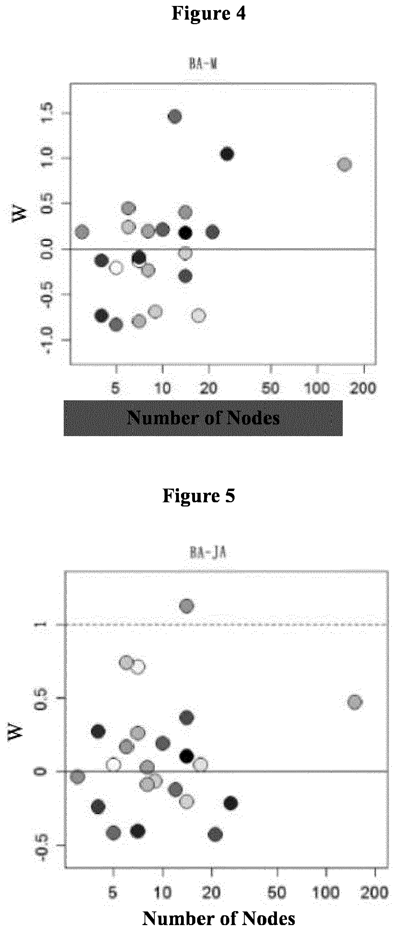

Totally 11 response modules in BA group were identified through comparing BA group and Vehicle group, with result shown in Fig. 4.

It was also found that there were differences between response modules of the groups intervened by different drugs compared with Vehicle group, indicating that the modules in the biological networks established after intervention by different drugs have topological differences in structure. Therefore, the method of the present invention can also be used for comparison of different drugs, and the module with a $\Delta t/T$ value greater than 0.2 was a differential module of the two groups. 9 differential modules were found through comparison of BA group and JA group, with result shown in Fig. 5.

5. Identification and comparison of drug target modules by function analysis

Function enrichment analysis of GO function and KEGG pathway in the response modules of three drug groups was carried out by utilizing function enrichment analysis tools (such as DAVIDE, etc.), in which the significance threshold for the enrichment analysis was set as p less than 0.05. Taking BA group as an example, among the 11 response modules in BA group, the most functions were enriched for BA-16 module, including 41 GO functions and 9 KEGG pathways, while BA-6 module was enriched no function. The biological functions enriched for BA-16 module are shown as follows.

| GO Function | P-value |
| --- | --- |
| GO:0007267∼cell-cell signaling | 0.001834725 |
| GO:0001890∼placenta development | 0.002565481 |
| GO:0006793∼phosphorus metabolic process | 0.005366167 |
| GO:0006796∼phosphate metabolic process | 0.005366167 |
| GO:0001701∼in utero embryonic development | 0.02229598 |
| GO:0042325∼ regulation of phosphorylation | 0.026015859 |
| GO:0019220∼ regulation of phosphate metabolic process | 0.027879898 |
| GO:0051174∼ regulation of phosphorus metabolic process | 0.027879898 |
| GO:0005216∼ ion channel activity | 0.032346557 |
| GO:0022838∼substrate specific channel activity | 0.034250297 |
| GO:0022803∼ passive transmembrane transporter activity | 0.035130286 |
| GO:0015267∼channel activity | 0.035130286 |
| GO:0000187∼ activation of MAPK activity | 0.043290991 |
| GO:0004714∼transmembrane receptor tyrosine kinase activity | 0.045409762 |
| **KEGG Pathway** | **P-value** |
| mmu05210: Colorectal cancer | 0.005863221 |
| mmu04010: MAPK signaling pathway | 0.049483325 |

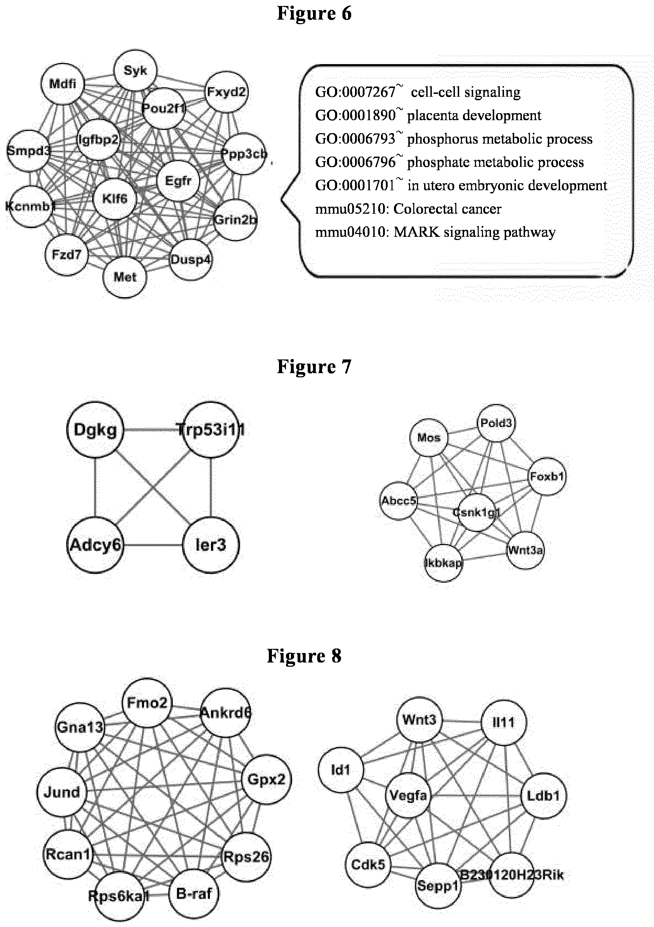

It is indicated that BA-16 module is related to biological functions such as cell signaling, placenta development, and phosphorus metabolic process, etc., as well as colon cancer and MAPK signaling pathway. Therefore, it can be concluded that the mechanism by which BA affects BA-16 response module is related to the above functions. Fig. 6 shows BA-16 module and functions enriched.

Biological function enrichment was carried out in all the obtained response modules of the three drug groups, BA group, JA group and UA group. It was found that, compared with the Vehicle group, 11 response modules were obtained from BA group, in which three of them failed to enrich any biological function and the other eight were target modules of BA; compared with the Vehicle group, 22 response modules were obtained from JA group, in which three of them failed to enrich any biological function and the other nineteen were target modules of JA; and compared with the Vehicle group, 8 response modules were obtained from UA group, in which one of them failed to enrich any biological function and the other seven were target modules of UA. The target modules of BA group (BA-5 and BA-15) were exemplified in Fig. 7.

Pairwise comparison was performed among BA, JA and UA groups, and if a module of one group was different from those of the other two groups, the module was identified as the characteristic module of the group. It was revealed that BA group had five characteristic modules after comparison, some of which (BA-9 and BA-21) were exemplified in Fig. 8; JA group had eight characteristic modules, while UA group had two characteristic modules.

Module related biological functions, which might be the action mechanism of the drugs, could be obtained through the function enrichment analysis of the modules.

**EXAMPLE 2** Verification of the identified drug target modules

1. Verification based on known knowledge of database

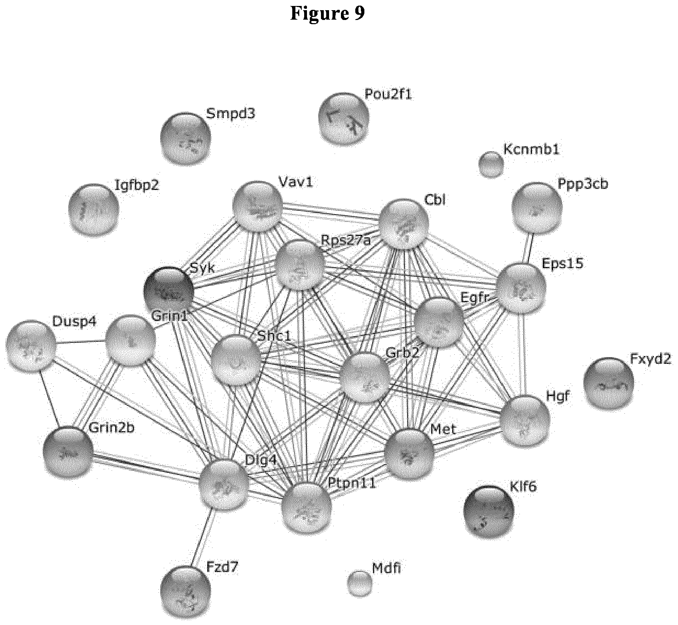

All known interactions between genes (proteins) in a module can be found through searching String (functional protein association networks) database. Taking BA-16 module as an example, the nodes in the module were input into the database, and interaction relationships of the nodes were obtained, which were shown in Fig. 9.

It can be seen from Fig. 9 that a number of nodes in the BA-16 module are connected by multiple edges, each edge representing one evidence type of relationship. Thus it is verified that interaction relationships do exist between those nodes.

2. Verification based on experiment

The significance of the composition of target modules was confirmed by gene expression levels through experimental method.

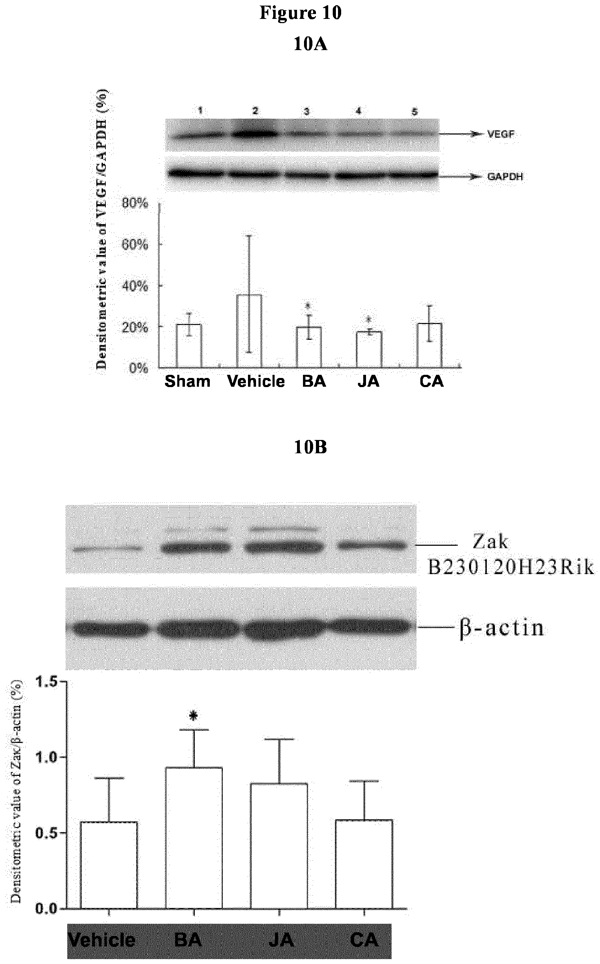

The genes in the target modules of each treatment group were verified by Western Blotting. In BA-21 module, the expression levels of VEGF and B230120H23Rik were significantly different from those in Vehicle group, which were shown in Fig. 10A and Fig. 10B respectively.

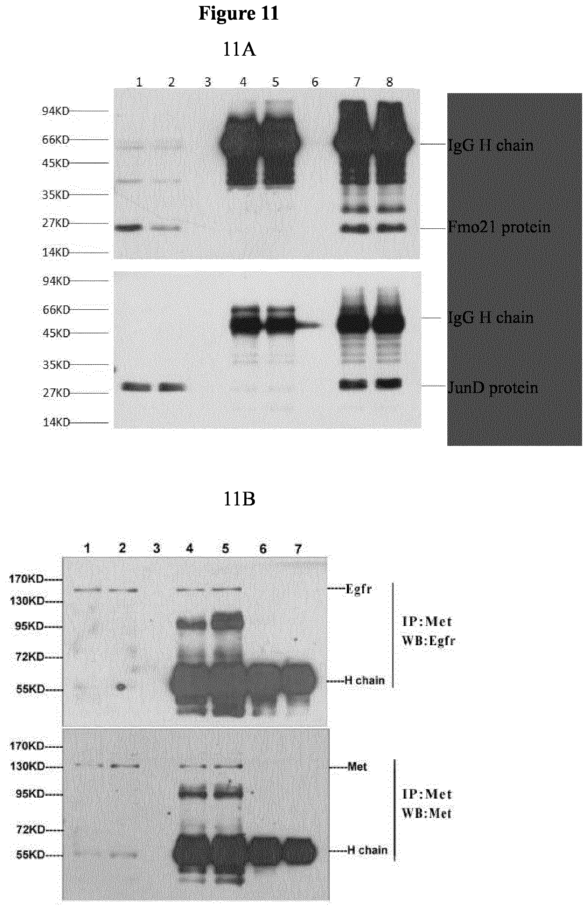

Co-Immunoprecipitation was used to confirm the interaction relationships between the proteins encoded by the genes in the modules. Results of Co-Immunoprecipitation of Met and Egfr proteins in BA-16 module demonstrated a direct interaction relationship between them (Fig. 11B), and results of Co-Immunoprecipitation of Fmo2 and Jund proteins in BA-9 module indicated a direct interaction relationship between them too (Fig. 11A).

**EXAMPLE 3** Application in gene expression network of anti PD-1 treatment of melanoma

In this Example, the method of the present invention was applied for mRNA expression data of anti-PD-1 treatment of melanoma, to identify the target modules of anti-PD-1 treatment of melanoma. Patients responding to anti-PD-1 therapy were divided into one group (effective group), while cases without response were divided into another group (control group), and gene co-expression networks of the two groups were constructed using WGCNA respectively.

Data used in this Example were downloaded from GEO (Gene Expression Omnibus) Database (https://www.ncbi.nlm.nih.gov/geo/ data ID: GSE78220). The data set is mRNA expression data of anti-PD-1 treatment of melanoma with samples from human, and from the data set 28 samples were selected, of which 15 cases belonged to the effective group and 13 cases belonged to the control group. Totally 25268 mRNAs were contained in the data. Specifically, implementing process is shown as follows:

1. Arrangement of the mRNA expression data

The downloaded data were arranged to a format of data table required by WGCNA analysis, in which the column of the table represented mRNA, while the row was the sample treated, and the numerical value in the corresponding cell was the expression value of the mRNA (row) in the sample (column). Network construction and module division were carried out based on mRNA co-expression.

2. Network construction and module division

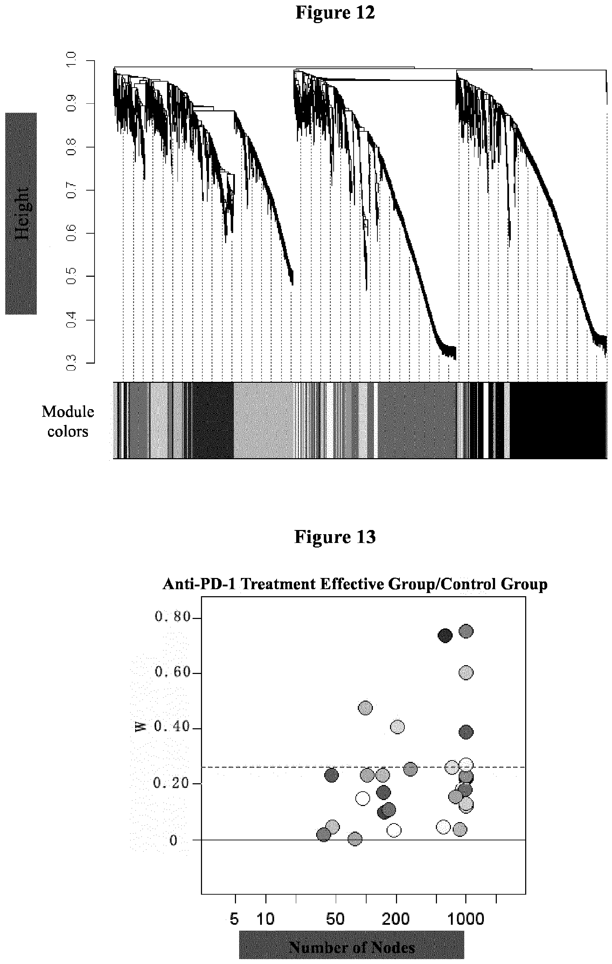

MRNA co-expression network of the effective group was constructed and accordingly divided into modules utilizing WGCNA as well. During network construction, 3080 mRNAs were removed due to excessive missing values, and the remaining 22188 mRNAs were used to construct the network (there were 21976 nodes in the network constructed) and to divide modules. Totally 30 modules were obtained from the effective group with module size ranging from 38 to 2900 nodes (mRNAs). Fig 12 shows the result of module division of the effective group by WGCNA.

3. Calculation of topological parameters of modules

Changes of topological characteristics of modules of effective group and control group were calculated. Density and connectivity were selected as topological indicators of the modules, and the density and connectivity of the modules of the effective group and the control group were calculated respectively. Comparison was made between the effective group and the control group to observe the changes in the indicators (density and connectivity) between the two groups.

4. Calculation of significance of the changes of topological characteristics

Significance of the changes of topological characteristics of the modules from the two groups was verified by bootstrap resampling. The genes comprised in the modules of the effective group were resampled 10000 times, the normalized $\Delta t/T$ value of each module compared with the control group was calculated, then accordingly the probability of normal distribution of the normalized $\Delta t/T$ values in the 10000 resamplings was obtained, and the correspondingly minimum value of the normalized $\Delta t/T$ values when statistical p value was less than or equal to 0.05 was the significance threshold of the change of topological parameters of the modules. When p was less than or equal to 0.05, W=0.26 was the significance threshold of the change of the modules. Compared with the control group, the module with a $\Delta t/T$ value greater than 0.26 in the anti PD-1 effective group could be identified as a drug response module.

Through comparison of the effective group and the control group, 8 response modules of anti PD-1 treatment of melanoma were obtained, with results shown in Fig. 13.

5. Identification of target modules of anti PD-1 treatment of melanoma by function analysis

Function enrichment analysis of GO function and KEGG pathway in the response modules of the effective group was carried out by utilizing function enrichment analysis tool (DAVIDE 6.7) with p less than 0.05 as the significance threshold of enrichment analysis. All of the 8 response modules of the anti PD-1 treatment effective group were enriched with corresponding functions, and were target modules of anti PD-1 treatment of melanoma.

**EXAMPLE 4** Application in gene expression network of infliximab treatment of rheumatoid arthritis (RA)

In this Example, the method of the present invention was applied for mRNA expression data of infliximab treatment of RA, to identify the target modules of infliximab treatment of RA utilizing 3 topological parameters (density, connectivity and entropy). Cases were divided into a control group (before infliximab treatment) and an effective group (after infliximab treatment), and gene co-expression networks of the two groups were constructed using WGCNA respectively.

Data used in this Example were downloaded from GEO (Gene Expression Omnibus) Database (https://www.ncbi.nlm.nih.gov/geo/, data ID: GSE8350). The data set is mRNA expression data of infliximab treatment of RA with samples from human, and from the data set 36 samples were selected for this Example, of which 18 cases belonged to the control group and the other 18 cases belonged to the effective group. Specifically, implementing process is shown as follows:

1. Arrangement of the mRNA expression data

The arrangement process was performed as described in EXAMPLE 3.

2. Network construction and module division

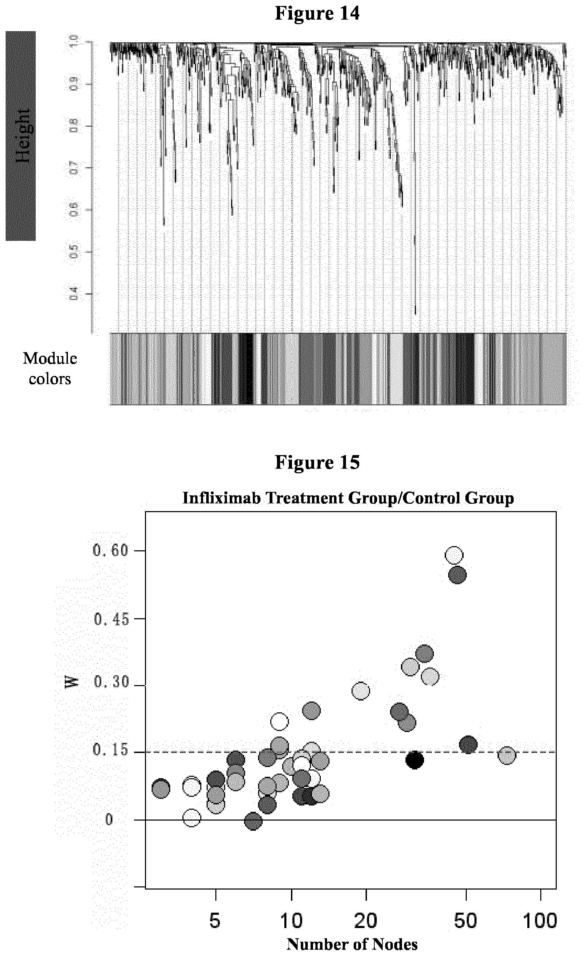

MRNA co-expression network of the effective group were constructed and accordingly divided into modules utilizing WGCNA as well. Totally 776 mRNAs were used to construct the network (there were 776 nodes in the network constructed) and to divide modules. As a result, 46 modules were obtained from the effective group with module size ranging from 3 to 73 nodes (mRNAs). Fig. 14 shows the result of module division of the effective group by WGCNA.

4. Calculation of topological parameters of modules

Changes of topological characteristics of modules of the effective group and control group were calculated. Density, connectivity and entropy were selected as topological indicators of the modules, and Density, connectivity and entropy of the modules of the effective group and the control group were calculated respectively. Comparison was made between the effective group and the control group to observe the changes in the indicators (density, connectivity and entropy) between the two groups.

4. Calculation of significance of the changes of topological characteristics

Significance of the changes of topological characteristics of the modules from the two groups was verified by Permutation test, in which the modules of the effective group were randomly permuted 1000 times, the normalized $\Delta t/T$ value of each module compared with the control group was calculated, then accordingly the probability of normal distribution of the normalized $\Delta t/T$ values in the 1000 random permutations was obtained, and the correspondingly minimum value of the normalized $\Delta t/T$ values when p was less than or equal to 0.05 was the significance threshold of the change of topological parameters of the modules. When p was less than or equal to 0.05, W=0.15 was the significance threshold of the change of the modules. Compared with the control group, the module with a $\Delta t/T$ value greater than 0.15 in the infliximab treatment group could be identified as a drug response module.

Through comparison of the effective group and the control group, 14 response modules of infliximab treatment of RA were obtained, with results shown in Fig. 15.

5. Identification of target modules of infliximab treatment of RA by function analysis

Function enrichment analysis of GO function and KEGG pathway in the response modules of the effective group was carried out by utilizing function enrichment analysis tool (DAVIDE 6.7) with p less than 0.05 as the significance threshold of enrichment analysis. 2 of the 14 response modules of the infliximab treatment of RA were enriched no biological functions, while the remaining 12 modules were the target modules of infliximab treatment of RA.

**EXAMPLE 5** Application in protein interaction network of mouse model of cerebral ischemia treated by baicalin

In this Example, the method of the present invention was applied for the protein interaction network in model mice of cerebral ischemia which were intervened with baicalin (BA), the bioactive ingredient extracted from Qingkailing. Compared with the Vehicle group, the differentially expressed genes of mice with cerebral ischemia intervened by BA were obtained, and were network mapped based on String database, then the protein interaction network of mice with cerebral ischemia intervened by BA was constructed accordingly. Then the method of the present invention was applied to identify the target modules of the protein interaction network of the mice with cerebral ischemia intervened by BA.

The difference between this Example and Example 1 lies in that the number of data sources utilized is different, and the network type of this Example is a protein interaction network. Therefore, both the results of module division and response module identification differ from those of Example 1. The following is only a description of what is different from Example 1.

1. Data of the protein interaction network

The differentially expressed genes of mice with cerebral ischemia intervened by BA were obtained, as described in EXAMPLE 1. Using String database, a protein interaction network comprising 2229 proteins (nodes) was constructed by mapping the differentially expressed genes.

2. Module division

Module division was carried out in the protein interaction network of mice with cerebral ischemia intervened by BA utilizing MCODE method, as a result 49 modules were obtained.

3. Calculation of topological parameters of modules

Changes of topological characteristics of the modules after BA intervention were calculated.

Density and connectivity were selected as topological indicators of the modules, and density and connectivity of the modules of the group intervened by BA and Vehicle group were calculated respectively. The modules comprised by the group intervened by BA were compared with the corresponding nodes in Vehicle group to observe the changes in the indicators (density and connectivity) between the two groups.

4. Calculation of significance of the changes of topological characteristics

Significance of the changes of topological characteristics of the modules from the two groups was verified by Permutation test, in which 49 modules of BA group were randomly permutated 1000 times, the normalized $\Delta t/T$ value of each module compared with the control group was calculated, then accordingly the probability of normal distribution of the normalized $\Delta t/T$ values in the 1000 random permutations was obtained, and the correspondingly minimum value

of the normalized $\Delta t/T$ values when p was less than or equal to 0.05 was the significance threshold of the change of topological parameters of the modules. When p was less than or equal to 0.05, W=0.11 was the significance threshold of the change of the modules. Compared with the Vehicle group, the module with a $\Delta t/T$ value greater than 0.11 in the group intervened by BA could be identified as a drug response module.

Through comparison of the BA group and the Vehicle group, 13 response modules were obtained.

5. Through function analysis, 6 response modules were enriched no biological functions, while the remaining 7 modules were the target modules of BA in the protein interaction network.

Through the analysis of the above Examples, it is proved that the present invention can effectively identify drug target modules in biological networks, and further obtain conservative modules and differential modules of different drugs by comparison of groups of different drug.

The above description of the specific embodiments of the present invention is not intended to limit the present invention. Person skilled in the art may make various changes or deformations according to the present invention, which shall fall within the scope of the claims appended to the present invention as long as they do not depart from the spirit of the present invention.

**Claims**

1. A method for identifying or comparing drug action module(s), comprising the following steps:

   (1) performing module division of a first biological network $G_i(V_i, E_i)$ and a second biological network $G_j(V_j, E_j)$ to obtain collections of module $G_i(M_i)$ and $G_j(M_j)$ respectively; wherein the first biological network $G_i(V_i, E_i)$ is a biological network before drug intervention, and the second biological network $G_j(V_j, E_j)$ is a biological network after drug intervention; or the first biological network $G_i(V_i, E_i)$ is a biological network after intervention by a first drug, and the second biological network $G_j(V_j, E_j)$ is a biological network after intervention by a second drug;
   (2) calculating one or more topological characteristics M(t) of each module in the collections of modules $G_i(M_i)$ and $G_j(M_j)$ respectively to obtain a single or combined topological indicator $T=M(t_1, t_2...t_m)$, wherein m is the number of the topological characteristics, and calculating a topological parameter change $\Delta t/T$ of corresponding modules in the twocollections of modules;
   (3) comparing the topological parameter change $\Delta t/T$ of the corresponding modules with a significance threshold W; when the first biological network $G_i(V_i, E_i)$ is a biological network before drug intervention and the second biological network $G_j(V_j, E_j)$ is a biological network after drug intervention, if $\Delta t/T$ is greater or equal to the significance threshold W, the module is a drug action module; when the first biological network $G_i(V_i, E_i)$ is a biological network after intervention by the first drug and the second biological network $G_j(V_j, E_j)$ is a biological network after intervention by the second drug, if $\Delta t/T$ is greater or equal to the significance threshold W, the module is a differential module between the actions of the first drug and the second drug.

2. The method according to claim 1, wherein the method further comprises:
   (4) performing biological function enrichment analysis of the drug action module to determine whether or not the module is a drug target module based on the results of function enrichment.

3. The method according to claim 1 or 2, wherein in step (1), the drug, the first drug and the second drug belong to the same type of traditional Chinese medicinal drug or chemical drug for prevention and treatment of disease; preferably, the first drug and the second drug are bioactive ingredients of the same type of traditional Chinese medicinal drug or chemical drug.

4. The method according to any one of claims 1 to 3, wherein in step (1), the first biological network and the second biological network are biological molecular networks, for example selected from the group consisting of gene co-expression network, protein interaction network, gene transcription regulatory network, non-coding gene regulatory network and metabolic network; more preferably, the first biological network and the second biological network are gene co-expression network or protein interaction network of the same type; more preferably, the number of nodes in the biological molecular network is greater or equal to 200, greater or equal to 500, greater or equal to 1000, or greater or equal to 5000.

5. The method according to any one of claims 1 to 4, wherein in step (1), module division is performed by utilizing a network analysis algorithm selected from the group consisting of clustering, heuristic search, seed extension, matrix decomposition, and topology network, etc.; more preferably, the module division is performed by utilizing a method selected from the group consisting of hierarchical clustering algorithm, WGCNA, MCODE, MCL, CFinder, CPM, SPC, G-N algorithm, ModuLand, DME, MINE, and SVD.

6. The method according to any one of claims 1 to 5, wherein in step (2), the topological characteristic M(t) is selected from the group consisting of modularity, degree, connectivity, density, clustering coefficient, betweenness, diameter, shortest path, entropy, and characteristic path length;
preferably, the number of the topological characteristics m is in the range of 1 to 10;
more preferably, the number of the topological characteristics m is 2, and the topological characteristic M(t) comprises density and connectivity; or preferably, the number of the topological characteristics m is 3, and the topological characteristic M(t) comprises density, connectivity and entropy.

7. The method according to any one of claims 1 to 6, wherein in step (2), calculating the topological parameter change $\Delta t/T$ comprises:
calculating the change of each topological parameter of each module of the first biological network compared with that of the corresponding module of the second biological network, i.e. calculating $\Delta t/T$ value of each topological parameter, and normalizing the $\Delta t/T$ value of each topological parameter by Min-max normalization, after which the $\Delta t/T$ value of each topological parameter is between (0, 1).

8. The method according to any one of claims 1 to 7, wherein in step (3), the significance threshold W of the change of topological parameter of module is calculated using permutation test or bootstrap resampling.

9. The method according to any one of claims 1 to 8, wherein in step (3), the significance threshold W is obtained by means as follow:
obtaining more than 1000 random modules corresponding to each module of the first biological network by utilizing permutation test or bootstrap resampling, calculating the normalized $\Delta t/T$ value of each random module compared with the corresponding module in the second biological network, and in turn calculating the probability of normal distribution of the normalized $\Delta t/T$ values of all random modules, accordingly the significance threshold W equaling the corresponding normalized $\Delta t/T$ value when p is less or equal to 0.05 in the probability of normal distribution.

10. The method according to any one of claims 1 to 9, wherein in step (4), the biological function enrichment analysis is performed through analyzing GO function and/or pathway.

# Figure 1

Biological Network after Drug Intervention

Module Division

Modules of Biological Network after Drug Intervention

Calculation of Topological Parameters

Topological Parameters of Each Module

Compared with Vehicle Group

Module Resampling

Certain Number of Random Modules

Compared with the Vehicle Group

Normalized $\Delta t/T$ Value of Change of Topological Parameters of Each Module

Normal Distribution of Change of $\Delta t/T$ Values

Determination of Threshold W

Drug Target Modules

Drug Response Modules

Function Enrichment

Drug Characteristic Modules

## Figure 2

## Figure 3

## Figure 4

BA-M

Number of Nodes

## Figure 5

BA-JA

Number of Nodes

## Figure 6

GO:0007267~ cell-cell signaling
GO:0001890~ placenta development
GO:0006793~ phosphorus metabolic process
GO:0006796~ phosphate metabolic process
GO:0001701~ in utero embryonic development
mmu05210: Colorectal cancer
mmu04010: MARK signaling pathway

## Figure 7

## Figure 8

# Figure 9

# Figure 10

## 10A

## 10B

# Figure 11

## 11A

## 11B

## Figure 12

Module
colors

## Figure 13

**Anti-PD-1 Treatment Effective Group/Control Group**

# Figure 14

# Figure 15

**Infliximab Treatment Group/Control Group**

# INTERNATIONAL SEARCH REPORT

| International application No. |
| --- |
| **PCT/CN2016/109895** |

### A. CLASSIFICATION OF SUBJECT MATTER

G06F 19/12 (2011.01) i

According to International Patent Classification (IPC) or to both national classification and IPC

### B. FIELDS SEARCHED

Minimum documentation searched (classification system followed by classification symbols)

G06F

Documentation searched other than minimum documentation to the extent that such documents are included in the fields searched

Electronic data base consulted during the international search (name of data base and, where practicable, search terms used)

CNKI, CNPAT, WPI, EPODOC, IEEE:compare, biological, drug, pharmacology, disease, network, topology, effect, module, difference, significance, level

### C. DOCUMENTS CONSIDERED TO BE RELEVANT

| Category* | Citation of document, with indication, where appropriate, of the relevant passages | Relevant to claim No. |
| --- | --- | --- |
| A | CN 103778349 A (SYSBIOMICS BIOINFORMATICS (BEIJING) CO., LTD.), 07 May 2014 (07.05.2014), description, paragraphs 0005 and 0033-0046, and figures 1-8 | 1-10 |
| A | CN 103782301 A (PHILIP MORRIS PRODUCTS S.A.), 07 May 2014 (07.05.2014), the whole document | 1-10 |
| A | CN 103514381 A (HUNAN UNIVERSITY), 15 January 2014 (15.01.2014), the whole document | 1-10 |
| A | WO 2015054266 A1 (THE REGENTS OF THE UNIVERSITY OF CALIFORNIA), 16 April 2015 (16.04.2015), the whole document | 1-10 |

☐ Further documents are listed in the continuation of Box C.  ☒ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 09 February 2017 (09.02.2017) | **08 March 2017 (08.03.2017)** |

| Name and mailing address of the ISA/CN: State Intellectual Property Office of the P. R. China No. 6, Xitucheng Road, Jimenqiao Haidian District, Beijing 100088, China Facsimile No.: (86-10) 62019451 | Authorized officer **ZHANG, Wen** Telephone No.:(86-10) **62413946** |
| --- | --- |

Form PCT/ISA/210 (second sheet) (July 2009)

## INTERNATIONAL SEARCH REPORT
### Information on patent family members

| International application No. |
| --- |
| **PCT/CN2016/109895** |

| Patent Documents referred in the Report | Publication Date | Patent Family | Publication Date |
| --- | --- | --- | --- |
| CN 103778349 A | 07 May 2014 | None | |
| CN 103782301 A | 07 May 2014 | EP 2754075 A2 | 16 July 2014 |
| | | JP 2014532205 A | 04 December 2014 |
| | | WO 2013034300 A2 | 14 March 2013 |
| | | US 2014214336 A1 | 31 July 2014 |
| | | HK 1197698 A0 | 06 February 2015 |
| CN 103514381 A | 15 January 2014 | None | |
| WO 2015054266 A1 | 16 April 2015 | US 2016246919 A1 | 25 August 2016 |

Form PCT/ISA/210 (patent family annex) (July 2009)